# EUROPÄISCHE PATENTSCHRIFT

(11) **EP 1 140 113 B1**
(45) Veröffentlichungstag und Bekanntmachung des Hinweises auf die Patenterteilung: **05.11.2003**
(21) Anmeldenummer: 99964669.8
(22) Anmeldetag: 23.12.1999
(51) Int. Cl.: A61K 31/663, A61P 31/00

(54) **VERWENDUNG VON BISPHOSPHONATEN ZUR PROPHYLAXE UND ZUR BEHANDLUNG VON INFEKTIÖSEN PROZESSEN**
USE OF BISPHOSPHONATES FOR THE PREVENTION AND TREATMENT OF INFECTIOUS PROCESSES
UTILISATION DE BISPHOSPHONATES POUR LA PROPHYLAXIE ET LE TRAITEMENT DE PROCESSUS INFECTIEUX

(30) Priorität: 23.12.1998 DE 19859668
(43) Veröffentlichungstag der Anmeldung: 10.10.2001
(73) Patentinhaber: BioAgency AG, 22525 Hamburg (DE)
(72) Erfinder: JOMAA, Hassan, D-35398 Giessen (DE)
(74) Vertreter: Dreiss, Fuhlendorf, Steimle & Becker
(86) Internationale Anmeldenummer: EP9910350
(87) Internationale Veröffentlichungsnummer: WO00038660

(56) Entgegenhaltungen:
- EP-A- 0 541 037
- WO-A-00/01238
- WO-A-97/43437
- DE-A- 19 828 450
- GB-A- 2 312 165
- US-A- 5 652 227
- BYINGTON, CARRIE L. ET AL: "Entamoeba histolytica: computer-assisted modeling of phosphofructokinase for the prediction of broad-spectrum antiparasitic agents" EXP. PARASITOL. (1997), 87(3), 194-202 , XP000911969
- NICOLAI B. ET AL: "[Also virus -induced Paget's disease can be treated]. EVENTUELL VIRAL BEDINGT, DENNOCH GUT BEHANDELBAR." THERAPIEWOCHE, (1995) 45/31 (1824+1826-1827). , XP000911950
- FAZIO G D ET AL: "ANTIVIRAL ACTIVITY OF 2 INORGANIC PYROPHOSPHATE ANALOGS PHOSPHONOACETIC ACID PAA AND TETRASODIUM CARBONYL BIPHOSPHONATE TCB" FITOPATOLOGIA BRASILEIRA, Bd. 13, Nr. 2, 1988, Seite 114 XP000861916 ISSN: 0100-4158
- SANSONI PAOLO ET AL: "Inhibition of antigen-presenting cell function by alendronate in vitro." JOURNAL OF BONE AND MINERAL RESEARCH, Bd. 10, Nr. 11, 1995, Seiten 1719-1725, XP000861815 ISSN: 0884-0431
- ZOJER N ET AL: "Effects of bisphosphonate treatment on lymphocyte subsets." ANNALS OF HEMATOLOGY, Bd. 77, Nr. SUPPL. 2, 1998, Seite S72 XP000861814 Annual Congress of the German and Austrian Societies of Hematology and Oncology;Frankfurt, Germany; October 25-28, 1998 ISSN: 0939-5555
- KUNZMANN V ET AL: "Stimulation of human gammadelta T cells by aminobisphosphonates used in treatment of bone disorders." BLOOD, Bd. 90, Nr. 10 SUPPL. 1 PART 1, 15. November 1997 (1997-11-15), Seite 575A XP000961891 39th Annual Meeting of the American Society of Hematology;San Diego, California, USA; December 5-9, 1997 ISSN: 0006-4971
- KUNZMANN V. ET AL: "Bisphosphonates used in treatment of bone disorders stimulate human.gamma..delta. T cells." IMMUNOBIOLOGY, (1997) 197/2-4 (221). , XP000911968
- KUNZMANN V ET AL: "Pamidronate induces gammadelta T cell expansion in vivo and mediates anti-plasma cell activity in multiple myeloma." BLOOD, Bd. 92, Nr. 10 SUPPL. 1 PART 1-2, 15. November 1998 (1998-11-15), Seite 279B XP000861909 40th Annual Meeting of the American Society of Hematology;Miami Beach, Florida, USA; December 4-8, 1998 ISSN: 0006-4971

## Beschreibung

Die Erfindung betrifft die Inaktivierung von γδ- T -Zellen durch die Verwendung von Bisphosphonaten zur therapeutischen und prophylaktischen Behandlung von Infektionen bei Mensch und Tier, die durch Viren, Bakterien, Pilze und Parasiten hervorgerufen werden.

Die Verwendung von Bisphosphonsäuren und einigen ihrer Derivate in Arzneimitteln ist bereits bekannt. Bislang ist die mikrobiostatische Wirksamkeit der Bisphosphonsäuren (DE 3 611 522), ihre Wirksamkeit bei der Behandlung von Störungen des Calcium- und Phosphatstoffwechsels (DE 2 534 390, DE 2 534 391, DE 3 334 211, DE 3 434 667, DE 2 745 083), die cytostatische Wirksamkeit (DE 3 425 812), ihre lipidsenkende Wirksamkeit (Arzneimittelforschung 46,759-62) und ihre Fähigkeit, Immunzellen zu stimulieren (WO 97/38696), bekannt.

Es besteht ein starker Bedarf, für die Bereicherung der Behandlung von Mensch und Tier Mittel bereitzustellen, die eine starke Wirksamkeit besitzen.

Aufgabe der vorliegenden Erfindung ist es daher, eine Substanz bereitzustellen, die bei Infektionen durch Viren, Bakterien, Pilze und Parasiten bei Menschen und Tieren einsetzbar ist und die oben angegebenen Bedingungen erfüllt.

Diese Aufgabe wird in völlig überraschender Weise durch die in Formel (I) definierte Stoffgruppe gelöst. Diese Stoffgruppe zeigt eine antiinfektiöse Wirkung gegen Viren, Bakterien, Pilze, ein- und mehrzellige Parasiten.

In US 5,652,227 werden Bisphosphonate zur Behandlung von Erkrankungen des Bindegewebes eingesetzt. Die Behandlung kann bei Säugetieren, einschließlich Menschen, erfolgen, die an einer exzessiven Matrix-Metallproteinase-Aktivität leiden, was zu einem extrazellulären Matrix-Proteinabbau führt. Ein extrazellulärer Matrix-Proteinabbau kann Teil des Erkrankungsverlaufs u.a. einer AIDS-Erkrankung oder Akne-Erkrankung sein. Als Beispiele für einsetzbare Bisphosphonate werden Clodronat, Etidronat, Pamidronat und Alendronat genannt.

In WO 00/01238 wird der Einsatz von Bisphosphonaten bei Erkrankungen von Menschen und Tieren, welche durch Nanobakterien verursacht werden, offenbart. Diese Nanobakterien tragen zu einer pathologischen Kalzifikation im menschlichen und tierischen Körper bei, wodurch Erkrankungen, wie z.B. Nierensteine verursacht werden. Zur Behandlung werden ein Antibiotikum, ein Bisphosphonat oder ein Kalziumchelatbildner entweder allein oder in Kombination in einer Menge verabreicht, die ausreicht, das Wachstum und die Entwicklung von Nanobakterien zu verhindern.

Das Immunsystem schützt Mensch und Tier vor Tumoren, Infektionen usw.. Bei der Konfrontation des Körpers mit einem Immunogen (z.B. Bestandteile eines Mikroorganismus) kommt es zu einer Vermehrung und Reifung von Zellen, welche dieses Immunogen abwehren können. Nur ein Teil des Immunsystems führt die eigentliche spezifische Immunreaktionen aus. Ein regulierender zweiter Teil leistet dazu Beihilfe. Die Immunsuppression gehört zu den Regulationselementen. Diese Zellen verhindern, daß die Immunreaktion bestimmte Grenzen nicht überschreitet. Bestimmte T-Zell-Populationen, wie z.B. die γδ-T-Zellen, können diese Immunsuppression ausführen (McMenamin et al., Science 1994 Sep. 23; 265(5180):1869-71). Diese Zellen werden von verschiedenen Mikroorganismen stimuliert (Jomaa et al. FEMS Immunol. Med. Microbiol. 1999 Sep.; 25(4); 371-8). Zu dieser Gruppe von Erregern gehören Plasmodium falciparum, Erreger der Malaria-, Mycobacterium tuberculosis, Erreger der Tuberkulose und das Eppstein-Barr-Virus, Erreger der Mononukleose. Diese Erreger halten durch Simulation immunosupprimierender γδ-T-Zellen das Immunsystem in Schach. Dadurch kommt keine echte Immunabwehr zustande. Die Mikroorganismen können dadurch im Wirt existieren und für sehr lange Zeit persistieren.

Es wurde nun gefunden, daß Stoffe der allgemeinen Formel (I) 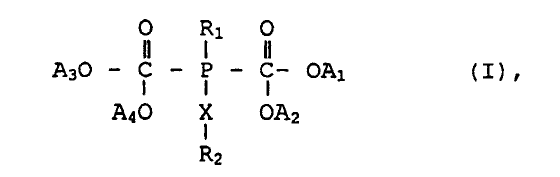 worin
A₁, A₂, A₃, A₄, die gleich oder verschieden sein können, aus der Gruppe ausgewählt sind, die aus Wasserstoff, Metallen der 1., 2. und 3. Hauptgruppe des Periodischen Systems, wie Na, K, Ca, Mg, Al sowie substituiertem und unsubstituiertem Ammonium und Ammoniumverbindungen, die sich von Ethylendiamin ableiten, besteht,
X wegfällt oder aus der Gruppe ausgewählt ist, die aus Alkylen mit bis zu 9 Kohlenstoffatomen, Alkenylen mit bis zu 9 Kohlenstoffatomen, Hydroxyalkylen mit bis zu 9 Kohlenstoffatomen und Amidino besteht,
R₁ aus der Gruppe ausgewählt ist, die aus H, OH, NH₂, -CH₃ besteht,
R₂ aus der Gruppe ausgewählt ist, die aus H, OH, -NH₂, substituiertem und unsubstituiertem Acyl, substituiertem und ursubstituiertem Alkyl, substituiertem und unsubstituiertem Aryl, substituiertem und unsubstituiertem Cycloalkyl, substituiertem und unsubstituiertem Aralkyl, substituiertem und unsubstituiertem heterocyclischen Rest besteht, und
deren pharmazeutisch verträgliche Salze, Amide, Ester sowie Salzen der Ester,
in Mensch und Tier zu einer Inaktivierung der γδ-T-Zellen führen. Damit eignen sich diese Stoffe zur Behandlung und Prophylaxe von Infektionskrankheiten, verursacht durch Parasiten, Viren und Bakterien mit Ausnahme von apatitbildenden Nanobakterien. Insbesondere eignen sich diese Stoffe für die Eradikationstherapie persistierender Infektionserreger einschließlich Helicobacter Pylori, Chlamydien und Hepatitis C Virus.

Ferner eignen sich diese Substanzen als Zusatz zu Impfstoffen zur Verstärkung der Immunreaktion bei Impfungen.

Bevorzugt eignen sich Stoffe der Formel (I), für die
A₁, A₂, A₃, A₄, die gleich oder verschieden sein können, aus der Gruppe ausgewählt sind, die aus Wasserstoff, den Metallen der 1., 2. oder 3. Hauptgruppe des Periodischen Systems, wie Na, K, Ca, Mg, Al substituiertem und unsubstituiertem Ammonium und Ammoniumverbindungen, die sich von Ethylendiamin oder Aminosäuren ableiten, besteht,
X wegfällt oder aus der Gruppe ausgewählt ist, die aus (CH₂)₁₋₆, insbesondere (CH₂)₁₋₅ und Amidino besteht,
R₁ aus der Gruppe ausgewählt ist, die aus H, OH, NH₂, -CH₃ besteht, und
R₂ aus der Gruppe ausgewählt ist, die aus -NH₂, 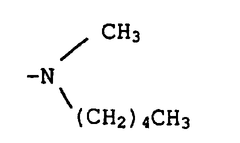 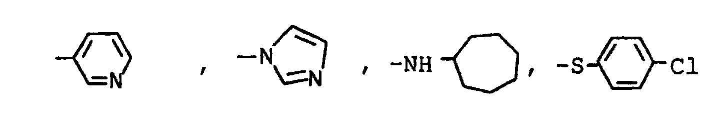 besteht.

Besonderheiten der obigen Definitionen und geeignete Beispiele dafür werden nachfolgend angegeben:
"Acyl" ist ein Substituent, der von einer Säure stammt, wie von einer organischen Carbonsäure, Kohlensäure, Carbaminsäure oder der den einzelnen vorstehenden Säuren entsprechenden Thiosäure oder Imidsäure, oder von einer organischen Sulfonsäure, wobei diese Säuren jeweils aliphatische, aromatische und/oder heterocyclische Gruppen im Molekül umfassen sowie Carbamoyl oder Carbamimidoyl.

Geeignete Beispiele für diese Acylgruppen werden nachfolgend angegeben.

Als aliphatische Acylgruppen werden von einer aliphatischen Säure stammende Acylreste bezeichnet, zu denen die folgenden gehören:
Alkanoyl (z.B. Formyl, Acetyl, Propionyl, Butyryl, Isobutyryl, Valeryl, Isovaleryl, Pivaloyl etc.); Alkenoyl (z. B. Acryloyl, Methacryloyl, Crotonoyl etc.); Alkylthioalkanoyl (z.B. Methylthioacetyl, Ethylthioacetyl etc.); Alkansulfonyl (z.B. Mesyl, Ethansulfonyl, Propansulfonyl etc.); Alkoxycarbonyl (z.B. Methoxycarbonyl, Ethoxycarbonyl, Propoxycarbonyl, Isopropoxycarbonyl, Butoxycarbonyl, Isobutoxycarbonyl etc.); Alkylcarbamoyl (z.B. Methylcarbamoyl etc.); (N-Alkyl)-thiocarbamoyl (z.B. (N-Methyl)-thiocarbamoyl etc.); Alkylcarbamimidoyl (z.B. Methylcarbamimidoyl etc.); Oxalo; Alkoxalyl (z.B. Methoxalyl, Ethoxalyl, Propoxalyl etc.).

Bei den obigen Beispielen für aliphatische Acylgruppen kann der aliphatische Kohlenwasserstoffteil, insbesondere die Alkylgruppe bzw. der Alkanrest, ggf. einen oder mehrere geeignete Substituenten aufweisen, wie Amino, Halogen (z.B. Fluor, Chlor, Brom etc.), Hydroxy, Hydroxyimino, Carboxy, Alkoxy (z.B. Methoxy, Ethoxy, Propoxy etc.), Alkoxycarbonyl, Acylamino (z.B. Benzyloxycarbonylamino etc.), Acyloxy (z.B. Acetoxy, Benzoyloxy etc.) und dergleichen; als bevorzugte aliphatische Acylreste mit solchen Substituenten sind z.B. mit Amino, Carboxy, Amino und Carboxy, Halogen, Acylamino oder dergleichen substituierte Alkanoyle zu nennen.

Als aromatische Acylreste werden solche Acylreste bezeichnet, die von einer Säure mit substituierter oder nicht substituierter Arylgruppe stammen, wobei die Arylgruppe Phenyl, Tolyl, Xylyl, Naphthyl und dergleichen umfassen kann; geeignete Beispiele werden nachfolgend angegeben:
Aroyl (z.B. Benzoyl, Toluoyl, Xyloyl, Naphthoyl, Phthaloyl etc.); Aralkanoyl (z.B. Phenylacetyl etc.); Aralkenoyl (z.B. Cinnamoyl etc.); Aryloxyalkanoyl (z.B. Phenoxyacetyl etc.); Arylthioalkanoyl (z.B. Phenylthioacetyl etc.); Arylaminoalkanoyl (z.B. N-Phenylglycyl, etc.); Arensulfonyl (z.B.Benzolsulfonyl, Tosyl bzw. Toluolsulfonyl, Naphthalinsulfonyl etc. ); Aryloxycarbonyl (z.B. Phenoxycarbonyl, Naphthyl-oxycarbonyl etc.); Aralkoxycarbonyl (z.B. Benzyloxycarbonyl etc.); Arylcarbamoyl (z.B. Phenylcarbamoyl, Naphthylcarbamoyl etc.); Arylglyoxyloyl (z.B. Phenylglyoxyloyl etc.).

Bei den vorstehenden Beispielen für aromatische Acylreste kann der aromatische Kohlenwasserstoffteil (insbesondere der Arylrest) und/oder der aliphatische Kohlenwasserstoffteil (insbesondere der Alkanrest) ggf. ein oder mehrere geeignete Substituenten aufweisen, wie solche, die als geeignete Substituenten für die Alkylgruppe bzw. den Alkanrest bereits angegeben wurden. Insbesondere und als Beispiel für bevorzugte aromatische Acylreste mit besonderen Substituenten werden mit Halogen und Hydroxy oder mit Halogen und Acyloxy substituiertes Aroyl und mit Hydroxy, Hydroxyimino, Dihalogenalkanoyloxyimino substituiertes Aralkanoyl angegeben sowie Arylthiocarbamoyl (z.B. Phenylthiocarbamoyl etc.); Arylcarbamimidoyl (z.B. Phenylcarbamimidoyl etc.).

Als heterocyclischer Acylrest wird ein Acylrest verstanden, der von einer Säure mit heterocyclischer Gruppe stammt; dazu gehören:
Heterocyclisches Carbonyl, bei dem der heterocyclische Rest ein aromatischer oder aliphatischer 5-bis 6-gliedriger Heterocyclus mit zumindest einem Heteroatom aus der Gruppe Stickstoff, Sauerstoff und Schwefel ist (z.B. Thiophen-yl, Furoyl, Pyrrolcarbonyl, Nicotinoyl etc.);
Heterocyclus-Alkanoyl, bei dem der heterocyclische Rest 5- bis 6-gliedrig ist und zumindest ein Heteroatom aus der Gruppe Stickstoff, Sauerstoff und Schwefel aufweist (z.B. Thiophenyl-acetyl, Furylacetyl, Imidazolylpropionyl, Tetrazolylacetyl, 2-(2-Amino-4-thiazolyl)-2-methoxyiminoacetyl etc.) und dergleichen.

Bei den obigen Beispielen für heterocyclische Acylreste kann der Heterocyclus und/oder der aliphatische Kohlenwasserstoffteil ggf. einen oder mehrere geeignete Substituenten aufweisen, wie die gleichen, die als geeignet für Alkyl- und Alkangruppen angegeben wurden.

"Alkyl" ist ein gerad- oder verzweigtkettiger Alkylrest mit bis zu 9 Kohlenstoffatomen, wie Methyl, Ethyl, Propyl, Isopropyl, Butyl, Isobutyl, tert.-Butyl, Pentyl, Hexyl und dergleichen.

Cycloalkyl steht vorzugsweise für ein ggfs. substituiertes C₃-C₇-Cycloalkyl; als mögliche Substituenten sind u.a. Alkyl, Alkoxy (z.B. Methoxy, Ethoxy etc.), Halogen (z.B. Fluor, Chlor, Brom etc.), Nitro und dergleichen geeignet.

Aryl ist ein aromatischer Kohlenwasserstoffrest, wie Phenyl Naphthyl usw., der ggf. einen oder mehrere geeignete Substituenten aufweisen kann wie Alkoxy (z.B. Methoxy, Ethoxy etc.), Halogen (z.B. Fluor, Chlor, Brom etc.), Nitro und dergleichen.

Zu "Aralkyl" gehören Mono-, Di-, Triphenylalkyle wie Benzyl, Phenethyl, Benzhydryl, Trityl und dergleichen, wobei der aromatische Teil ggf. ein oder mehrere geeignete Substituenten aufweisen kann wie Alkoxy (z.B. Methoxy, Ethoxy etc.), Halogen (z.B. Fluor, Chlor, Brom etc.), Nitro und dergleichen.

Zu "Alkylen" gehören gerad- oder verzweigtkettige Alkylengruppen, die bis zu 9 Kohlenstoffatome aufweisen und durch die Formel

-(CₙH₂ₙ)-

wiedergegeben werden können, in der n eine ganze Zahl von 1 bis 9 ist, wie Methylen, Ethylen, Trimethylen, Methylethylen, Tetramethylen, 1-Methyltrimethylen, 2-Ethylethylen, Pentamethylen, 2-Methyltetramethylen, Isopropylethylen, Hexamethylen, und dergleichen; bevorzugte Alkylenreste haben bis zu 4 Kohlenstoffatome und besonders bevorzugt sind Reste mit 3 Kohlenstoffatomen wie z.B. Trimethylen.

Zu "Alkenylen" gehören gerad- oder verzweigtkettige Alkenylengruppen mit bis zu 9 Kohlenstoffatomen, die durch die Formel

-(CₙH₂ₙ₋₂)-

wiedergegeben werden können, in der n eine ganze Zahl von 2 bis 9 ist, wie z.B. Vinylen, Propenylen (z.B. 1-Propenylen, 2-Propenylen), 1-Methylpropenylen, 2-Methylpropenylen, Butenylen, 2-Ethylpropenylen, Pentenylen, Hexenylen und dergleichen; besonders bevorzugt kann der Alkenylenrest bis zu 5 Kohlenstoffatome aufweisen und insbesondere 3 Kohlenstoffatome wie z.B. 1-Propenylen.

Zu "Hydroxyalkylen" gehören gerad- oder verzweigtkettige Alkylenreste, die bis zu 9 Kohlenstoffatome aufweisen, wobei ein oder mehrere ausgewählte Kohlenstoffatome mit einer Hydroxygruppe substituiert ist; diese Reste können durch die Formel

-(CₙH_{2n-z})(OH)_{z}-

wiedergegeben werden, in der n eine ganze Zahl von 1 bis 9 ist und z eine ganze Zahl von 1 bis 9 ist, für die z ≤ n gilt. Zu geeigneten Beispielen für solche Hydroxyalkylengruppen gehören Hydroxymethylen, Hydroxyethylen (z.B. 1-Hydroxyethylen und 2-Hydroxy-ethylen), Hydroxytrimethylen (z.B. 1-Hydroxytrimethylen, 2-Hydroxytrimethylen und 3-Hydroxytrimethylen), Hydroxytetra-methylen (z.B. 2-Hydroxytetramethylen), 2-Hydroxy-2-methyltri-methylen, Hydroxypentamethylen (z.B. 2-Hydroxypentamethylen), Hydroxyhexamethylen (z.B. 2-Hydroxyhexamethylen) und dergleichen. Besonders bevorzugt wird ein niederes Hydroxyalkylen mit bis zu 4 Kohlenstoffatomen und insbesondere ein solches mit 3 Kohlenstoffatomen wie z.B. 2-Hydroxytrimethylen.

"Heterocyclischer Rest" ist vorzugsweise ein aromatischer oder aliphatischer 5-bis 6-gliedriger Heterocyclus mit zumindest einem Heteroatom aus der Gruppe Stickstoff, Sauerstoff und Schwefel (z.B. Thiophenyl, Furoyl, Pyrrolcarbonyl, Nicotinoyl etc.).

Als besonders wirksam haben sich die Bisphosphonsäuren
Amino-hydroxy-methyliden-bisphosphonsäure (AMP),
2-Amino-1-hydroxyethyliden-1,1-bisphosphonsäure (AEP),
3-Amino-1-hydroxypropyliden-1,1-bisphosphonsäure (Pamidronsäure),
4-Amino-1-hydroxybutyliden-1,1-bisphosphonsäure (Alendronsäure),
6-Amino-1-hydroxyhexyliden-1,1-bisphosphonsäure (AHP),
Amidinomethylen-bisphosphonsäure (AIMP),
3-Methylpentylamino-1-hydroxypropyliden-1,1-bisphosphonsäure (Ibandronsäure),
2-(3-Pyridinyl)-1-hydroxyethyliden-bisphosphonsäure (Risedronsäure),
1-Hydroxy-2-(imidazol-1-yl)-ethyliden-1,1-bisphosphonsäure (Zoledronsäure),
Cycloheptylaminomethylendiphosphonsäure (Cimadronsäure),
4-Chlorphenyl-thiomethylen-1,1-bisphosphonsäure (Tiludronsäure) sowie deren Derivate erwiesen.

Die Verbindungen sind insbesondere für die therapeutische und prophylaktischen Behandlung von Infektionen bei Mensch und Tier geeignet, die durch Viren, Bakterien, ein- und mehrzellige Parasiten und Pilze hervorgerufen werden.

Die Bisphosphonsäuren und ihre Derivate eigenen sich zur Behandlung der Acne vulgaris, der Tuberkulose bei Mensch und Tier, der Lepra, und weiterer Mykobacteriosen bei Mensch und Tier, der Paratuberkulose der Tiere, von Campylobacter-Enteritiden bei Mensch und Tier.

Weiter ist der Einsatz besonderes bei der Helicobacter-Eradikationstherapie bei Ulcera des Magendarmtraktes bevorzugt.

Die Substanzen eignen sich ferner insbesondere für die Eradikationstherapie der Chlamydien zur Vorbeugung oder im Rahmen einer Therapie von Herz- und Gefäßerkrankungen, insbesondere der koronaren Herzkrankheit.

Es können auch Kombination mit einem weiteren Antibiotikum zur Behandlung der obengenannten Erkrankungen eingesetzt werden. Für Kombinationspräparate mit anderen Antiinfektiva eignen sich insbesondere Isoniazid, Rifampicin, Ethambutol, Pyrazinamid, Strepromycin, Protionamid und Dapson zur Behandlung der Tuberkulose.

Die erfindungsgemäßen Bisphosphonate sind zur Bekämpfung folgender viraler Infekte geeignet:
Eradikation von Papillomaviren zur Vorbeugung von Tumoren, insbesondere von Tumoren der Geschlechtsorganen verursacht durch Papillomaviren beim Menschen, Eradikation von Herpesviren, Eradikation humaner Herpesviren 8 zur Behandlung der Kaposi-Sarkoma, Eradikation von Zytomegalie-Viren vor Transplantationen, Eradikation von Eppstein-Barr-Viren vor Transplantation und zur Vorbeugung von Eppstein-Barr-Viren-assozierten Tumoren, Eradikation von Hepatitisviren zur Behandlung von chronischen Leber-Erkrankungen und zur Vorbeugung von Lebertumoren und Leberzirrhosen, Eradikation von Coxsackieviren bei Kardiomyopathien, Eradikation von Coxsackieviren bei Diabetes-mellitus-Patienten, Eradikation von Immunschwäche-Viren in Mensch und Tier, Behandlung von Begleitinfektionen in AIDS-Patienten, Behandlung von Entzündungen viraler Genese des Respirationstraktes (Larynxpapillome, Hyberplasien, Rhinitis, Pharyngitis, Bronchitis, Pneumonien), der Leber und des Gallensystems (Hepatitis, Cholangitis, hepatozelluläres Karzinom), des lymphatischen Gewebes (Mononukleose, Lymphadenitis), des hämatopoetischen Systems, der Haut (Warzen, Dermatitis, Herpes labialis, Fieberbläschen, Herpes Zoster, Gürtelrose), der Schleimhäute (Papillome, Konjunktivapapillome, Hyperplasien, Dysplasien), des Herz-Blutgefäß-Systems (Arteriitis, Myokarditis, Endokarditis, Perikarditis), des Nieren-Harnweg-Systems, der Geschlechtsorgane (Anogenitale Läsionen, Warzen, Genitalwarzen, spitzen Kondylome, Dysplasien, Papillome, Zervixdysplasien, Condylomata acuminata, Epidermodysplasia verruciformis), der Bewegungsorgane (Myositis, Myalgien).

Die Mittel können in Kombination mit anderen Mitteln mit antiviralen Eigenschaften eingesetzt werden.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt. Tabletten, Dragees, Kapseln, Pillen und Granulate können den oder die Wirkstoffe neben den üblichen Trägerstoffen enthalten, wie (a) Füllund Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. C₁₄-Alkohol mit C₁₆-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z. B. Pfefferminzöl und Eukalyptusöl und Süßmittel, z. B. Saccharin, enthalten.

Bisphosphonsäuren und ihre Derivate der Formel (I) sollen in den oben aufgeführten pharmazeutischen Zubereitungen, vorzugsweise in einer Konzentration von etwa 0,1 bis 99,5 Gew.-%, vorzugsweise von etwa 0,5 bis 95 Gew.-%, der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracistemal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionslösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Die notwendige Menge der einzelnen Derivate zur Erzielung des gewünschten Effektes unterscheiden sich sehr stark. Im allgemeinen hat es sich sowohl in der Human- als auch in der Veterinärmedizin als vorteilhaft erwiesen, den oder die Bisphosphonate der Formel (I) in Gesamtmengen von etwa 0,005 bis etwa 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 0,002 bis etwa 50 mg/kg Körpergewicht. Es kann jedoch erforderlich sein, von den genannten Dosierungen abzuweichen, und zwar in Abhängigkeit von der Art und dem Körpergewicht des zu behandelnden Patienten, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch den Fachmann aufgrund seines Fachwissens erfolgen.

Bei der Behandlung von Tieren können die erfindungsgemäß zu verwendenden Verbindungen in den üblichen Konzentrationen und Zubereitungen zusammen mit dem Futter bzw. mit Futterzubereitungen oder mit dem Trinkwasser gegeben werden.

Im folgenden sind einige Beispiele zur Wirksamkeit aufgeführt:

### Beispiel 1

Gesunden Probanden wurde im Abstand von jeweils zwei Wochen 90 mg Pamidonsäure als Infusion verabreicht. Nach der 3. Infusion wurde den Probanden Blut entnommen. Aus dem Blut wurden die mononukleären Zellen aufgereinigt. Die Aktivierbarkeit der γδ-T-Zellen wurde nun getestet. Die genaue Beschreibung ist in Jomaa et al. FEMS Immunol. Med. Microbiol. 1999 Sep.; 25(4); 371-8 veröffentlicht.

Die Zellen der behandelten Probanden zeigen keine γδ-T-Zellen-Aktivierung durch aus Mikroorganismen gewonnenen Antigenen. Im Gegensatz dazu ließen sich die Zellen von Kontrollpersonen aktivieren.

### Beispiel 2

Die Zellen von Probanden, die entsprechend der Vorgehensweise aus Beispiel 1mit Ibandronsäure (1 mg pro Behandlung) behandelt wurden, zeigten keine γδ-T-Zellen-Aktivierung durch Antigene, die aus Mikroorganismen gewonnen wurden.

### Beispiel 3

Die Zellen von Probanden, die entsprechend der Vorgehensweise aus Beispiel 1 und 2 mit Zoledronsäure behandelt wurden, zeigten keine γδ-T-Zellen-Aktivierung durch Antigene, die aus Mikroorganismen gewonnen wurden.

## Patentansprüche

1. Verwendung von Bisphosphonsäuren der allgemeinen Formel 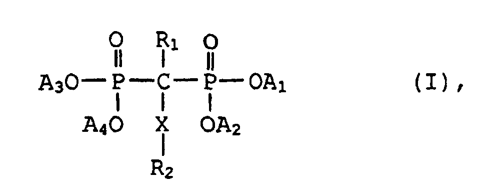 worin
A₁, A₂, A₃, A₄, die gleich oder verschieden sein können, aus der Gruppe ausgewählt sind, die aus Wasserstoff, Metallen der 1., 2. und 3. Hauptgruppe des periodischen Systems, wie Na, K, Ca, Mg, Al sowie substituiertem und unsubstituiertem Ammonium und Ammoniumverbindungen, die sich von Ethylendiamin ableiten, besteht,
X wegfällt oder aus der Gruppe ausgewählt ist, die aus Alkylen mit bis zu 9 Kohlenstoffatomen, Alkenylen mit bis zu 9 Kohlenstoffatomen, Hydroxyalkylen mit bis zu 9 Kohlenstoffatomen und Amidino besteht,
R₁ aus der Gruppe ausgewählt ist, die aus H, OH, NH₂, -CH₃ besteht,
R₂ aus der Gruppe ausgewählt ist, die aus H, OH, -NH₂, substituiertem und unsubstituiertem Acyl, substituiertem und unsubstituiertem Alkyl, substituiertem und unsubstituiertem Aryl, substituiertem und unsubstituiertem Cycloalkyl, substituiertem und unsubstituiertem Aralkyl, substituiertem und unsubstituiertem heterocyclischen Rest besteht, und
deren pharmazeutisch verträglichen Salzen, Amiden, Estern sowie Salzen der Ester zur Herstellung von Arzneimitteln zur Inaktivierung der γδ-T-Zellen zur Vorbeugung und Therapie von Erkrankungen, welche durch Parasiten, Viren, Pilze und Bakterien mit Ausnahme von apatitbildenden Nanobakterien verursacht sind, mit Ausnahme von AIDS und durch AIDS ausgelöste Entzündungen sowie deren Folgeerkrankungen, nämlich der Degeneration von Bindegewebe.

2. Verwendung nach Anspruch 1, **dadurch gekennzeichnet, daß**
A₁, A₂, A₃, A₄, die gleich oder verschieden sein können, aus der Gruppe ausgewählt sind, die aus Wasserstoff, den Metallen der 1., 2, oder 3. Hauptgruppe des periodischen Systems, wie Na, K, substituiertem und unsubstituiertem Ammonium und Ammoniumverbindungen, die sich von Ethylendiamin ableiten, besteht,
X wegfällt oder aus der Gruppe ausgewählt ist, die aus (CH₂)₁₋₆, insbesondere (CH₂)₁₋₅ und Amidino besteht,
R₁ aus der Gruppe ausgewählt ist, die aus H, OH, NH₂, -CH₃ besteht, und
R₂ aus der Gruppe ausgewählt ist, die aus -NH₂, 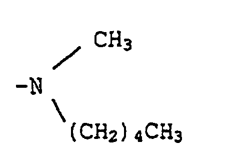 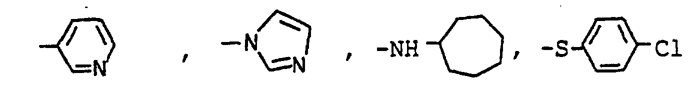 besteht

3. Verwendung nach Anspruch 2,
**dadurch gekennzeichnet, daß**
die Bisphosphonate aus der Gruppe ausgewählt sind, die aus Aminohydroxymethylidenbisphosphonsäure,
2-Amino-1-hydroxyethyliden-1,1-bisphosphonsäure,
3-Amino-1-hydroxypropyliden-1,1-bisphosphonsäure,
4-Amino-1-hydroxybutyliden-1,1-bisphosphonsäure,
6-Amino-1-hydroxyhexyliden-1,1-bisphosphonsäure,
Amidinomethylen-bisphosphonsäure,
3-Methylpentylamino-1-hydroxypropyliden-1,1-bisphosphonsäure,
2-(3-Pyridinyl)-1-hydroxyethyliden-bisphosphonsäure,
1-Hydroxy-2-(imidazol-1-yl)-ethyliden-1,1-bisphosphonsäure,
Cycloheptylaminomethylendiphosphonsäure,
4-Chlorphenyl-thiomethylen-1,1-bisphosphonsäure sowie deren Derivaten besteht.

4. Verwendung nach einem der vorhergehenden Ansprüche zur Therapie und Prophylaxe der Acne vulgaris, der Tuberkulose bei Mensch und Tier, der Lepra, und weiterer Mykobacteriosen bei Mensch und Tier, der Paratuberkulose der Tiere, von Campylobacter-Enteritiden bei Mensch und Tier, der Helicobacter Pylori und der Chlamydien zur Vorbeugung oder im Rahmen einer Therapie von Herz- und Gefäßerkrankungen, insbesondere der koronaren Herzkrankheit.

5. Verwendung nach einem der Ansprüche 1 bis 3 bei der Eradikationstherapie von Bakterien und Viren.

6. Verwendung nach Anspruch 5 zur Eradikation von Helicobacter Pylori und Chlamydien.

7. Verwendung nach Anspruch 5 zur Eradikation von Papillomaviren zur Vorbeugung von Tumoren, insbesondere von Tumoren der Geschlechtsorganen verursacht durch Papillomaviren beim Menschen, Eradikation von Herpesviren, Eradikation humaner Herpesvi ren 8 zur Behandlung der Kaposi-Sarkoma, Eradikation von Zytomegalie-Viren vor Transplantationen, Eradikation von Eppstein-Barr-Viren vor Transplantation und zur Vorbeugung von Eppstein-Barr-Viren-assozierten Tumoren, Eradikation von Hepatitisviren zur Behandlung von chronischen Leber-Erkrankungen und zur Vorbeugung von Lebertumoren und Leberzirrhosen, Eradikation von Coxsackieviren bei Kardiomyopathien, Eradikation von Coxsackieviren bei Diabetes-mellitus-Patienten, Eradikadon von Immunschwäche-Viren in Mensch und Tier, Behandlung von Begleitinfektionen in AIDS-Patienten, Behandlung von Entzündungen viraler Genese des Respirationstraktes (Larynxpapillome, Hyberplasien, Rhinitis, Pharyngitis, Bronchitis, Pneumonien), der Leber und des Gauensystems (Hepatitis, Cholangitis, hepatozelluläres Karzinom), des lymphatischen Gewebes (Mononukleose, Lymphadenitis), des hämatopoetischen Systems, der Haut (Warzen, Dermatitis, Herpes labialis, Fieberbläschen, Herpes Zoster, Gürtefrose), der Schleimhäute (Papillome, Konjunktivapapillome, Hyperplasien, Dysplasien), des Herz-Blutgefäß-Systems (Arteriitis, Myokarditis, Endokarditis, Perikarditis), des Nieren-Harnweg-Systems, der Geschlechtsorgane (Anogenitale Läsionen, Warzen, Genitalwarzen, spitzen Kondylome, Dysplasien, Papillome, Zervixdysplasien, Condylomata acuminata, Epidermodysplasia verruciformis), der Bewegungsorgane (Myositis, Myalgien) mit Ausnahme von AIDS und durch AIDS ausgelöste Entzündungen sowie deren Folgeerkrankungen, nämlich die Degeneration von Bindegewebe.

8. Verwendung nach Anspruch 7 zur Eradikation des Hepatitis C Virus.

9. Verwendung nach einem der vorhergehenden Ansprüche, **dadurch gekennzeichnet, daß** das Arzneimittel ferner einen pharmazeutisch akzeptablen Träger enthält.

10. Verwendung nach einem der vorhergehenden Ansprüche als Zusatz zu Impfstoffen.

## Claims

1. Use of bisphosphonic acids of the general formula 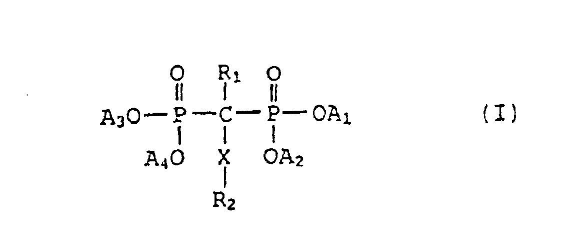 wherein
A₁, A₂, A₃, A₄, which can be the same or different, are selected from the group which comprises hydrogen, metals of the 1^{st}, 2^{nd} and 3^{rd} main group of the periodic table, such as Na, K, Ca, Mg, Al and substituted and unsubstituted ammonium and ammonium compounds which are derived from ethylenediamine,
X is omitted or is selected from the group which comprises alkylene with up to 9 carbon atoms, alkenylene with up to 9 carbon atoms, hydroxyalkylene with up to 9 carbon atoms and amidino,
R₁ is selected from the group which comprises H, OH, NH₂, -CH₃,
R₂ is selected from the group which comprises H, OH, -NH₂, substituted and unsubstituted acyl, substituted and unsubstituted alkyl, substituted and unsubstituted aryl, substituted and unsubstituted cycloalkyl, substituted and unsubstituted aralkyl, substituted and unsubstituted heterocyclic radical,
and
the pharmaceutically tolerated salts thereof, amides, esters and salts of the esters for the production of medicines for the inactivation of γδ T cells for the prevention and therapy of diseases which are caused by parasites, viruses, fungi and bacteria with the exception of apatite-forming nanobacteria, with the exception of AIDS and inflammations triggered by AIDS and also consequential diseases thereof, namely the degeneration of connective tissue.

2. Use according to claim 1, **characterised in that**
A₁, A₂, A₃, A₄, which can be the same or different, are selected from the group which comprises hydrogen, the metals of the 1^{st}, 2^{nd} or 3^{rd} main group of the periodic table, such as Na, K, substituted and unsubstituted ammonium and ammonium compounds which are derived from ethylenediamine,
X is omitted or is selected from the group which comprises (CH₂)₁₋₆, in particular (CH₂)₁₋₅ and amidino,
R₁ is selected from the group which comprises H, OH, NH₂, -CH₃, and
R₂ is selected from the group which comprises NH₂,

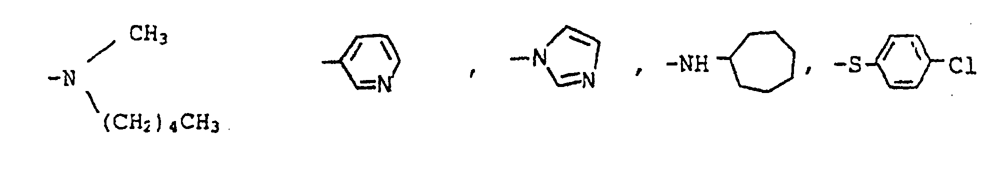

3. Use according to claim 2,
**characterised in that**
the bisphosphonates are selected from the group which comprises aminohydroxymethylidene bisphosphonic acids,
2-amino-1-hydroxyethylidene-1,1-bisphosphonic acid,
3-amino-1-hydroxypropylidene 1,1-bisphosphonic acid,
4-amino-1-hydroxybutylidene-1,1-bisphosphonic acid,
6-amino-1-hydroxyhexylidene-1,1-bisphosphonic acid,
amidinomethylene-bisphosphonic acid,
3-methylpentylamino-1-hydroxypropylidene-1,1-bisphosphonic acid,
2-(3-pyridinyl)-1-hydroxyethylidene-bisphosphonic acid,
1-hydroxy-2-(imidazol-1-yl)-ethylidene-1,1-bisphosphonic acid,
cycloheptylaminomethylene diphosphonic acid,
4-chlorophenyl-thiomethylene-1,1-bisphosphonic acid and derivatives thereof.

4. Use according to one of the preceding claims for the therapy and prophylaxis of acne vulgaris, human and animal tuberculosis, leprosy and further human and animal mycobacterioses, paratuberculosis in animals, campylobacter enteritides in humans and animals, helicobacter pylori, and chlamydiae for the prevention of or within the framework of a therapy for coronary and vascular diseases, in particular coronary heart disease.

5. Use according to one of the claims 1 to 3 in the eradication therapy of bacteria and viruses.

6. Use according to claim 5 for the eradication of helicobacter pylores and chlamydiae.

7. Use according to claim 5 for the eradication of papilloma viruses for the prevention of tumours, in particular tumours of the sexual organs caused by papilloma viruses in humans, eradication of herpes viruses, eradication of human herpes viruses 8 for the treatment of Kaposi's sarcoma, the eradication of cytomegalic viruses before transplantations, eradication of Epstein-Barr viruses before transplantation and for preventing tumours associated with Epstein-Barr viruses, eradication of hepatitis viruses for the treatment of chronic liver diseases and for the prevention of liver tumours and liver cirrhoses, eradication of coxsackie viruses in cardiomyopathies, eradication of coxsackie viruses in diabetes mellitus patients, eradication of immunodeficiency viruses in humans and animals, treatment of accompanying infections in AIDS patients, treatment of inflammations of viral genesis of the respiratory tract (larynx papilloma, hyperplasias, rhinitis, pharyngitis, bronchitis, pneumonias), of the liver and of the gall bladder system (hepatitis, cholangitis, hepatocellular carcinoma), of the lymphatic tissue (mononucleosis, lymphadenitis), of the haematopoietic system, of the skin (warts, dermatitis, herpes labialis, fever blisters, herpes zoster, shingles), of the mucus membranes (papilloma, conjunctiva papilloma, hyperplasias, dysplasias), of the cardiovascular system (arteritis, myocarditis, endocarditis and pericarditis), of the renal-urinary tract system, of the sexual organs (anogenital lesions, warts, genital warts, acute condyloma, dysplasias, papilloma, cervical dysplasias, condylomata acuminata, epidermodysplasia verruciformis), of the motor organs (myositis, myalgias) with the exception of AIDS and inflammations triggered by AIDS and also the consequential diseases thereof, namely the degeneration of connective tissue.

8. Use according to claim 7 for the eradication of the hepatitis C virus.

9. Use according to one of the preceding claims, **characterised in that** the medicine contains furthermore a pharmaceutically acceptable carrier.

10. Use according to one of the preceding claims as an additive in vaccines.

## Revendications

1. Utilisation d'acides biphosphoniques de formule générale 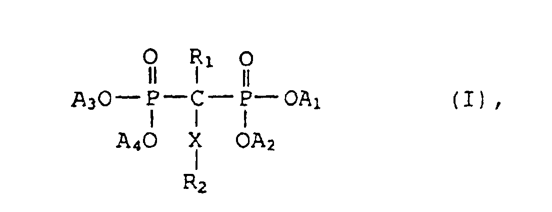 dans laquelle
A₁, A₂, A₃ et A₄, pouvant être identiques ou différents, sont choisis dans le groupe constitué par l'hydrogène, les métaux des groupes principaux 1, 2 et 3 de la Classification Périodique des Eléments tels que Na, K, Ca, Mg, Al ainsi que l'ammonium substitué ou non substitué et les composés combinés à l'ammonium dérivés de l'éthylènediamine,
X est une liaison simple ou bien est choisi dans le groupe constitué par un groupe alcène possédant jusqu'à 9 atomes de carbone, un groupe alcényle possédant jusqu'à 9 atomes de carbone, un groupe hydroxyalcène possédant jusqu'à 9 atomes de carbone et un groupe amidino,
R₁ est choisi dans le groupe constitué par H, OH, NH₂, -CH₃,
R₂ est choisi dans le groupe constitué par H, OH, -NH₂, un groupe acyle substitué ou non substitué, un groupe alkyle substitué ou non substitué, un groupe aryle substitué ou non substitué, un groupe cycloalkyle substitué ou non substitué, un groupe aralkyle substitué ou non substitué, un groupe hétérocyclique substitué ou non substitué,
et
de leurs sels, de leurs amides, de leurs esters acceptables sur le plan pharmaceutique ainsi que des sels des esters pour la préparation de médicaments destinés à l'inactivation des cellules T γδ en vue de la prophylaxie et de la thérapie de maladies causées par des parasites, des virus, des champignons et des bactéries, à l'exception des nanobactéries formant de l'apatite, à l'exception du SIDA et des inflammations provoquées par le SIDA ainsi que de ses maladies opportunistes, à savoir la dégénérescence des tissus conjonctifs.

2. Utilisation selon la revendication 1, **caractérisée en ce que**
A₁, A₂, A₃ et A₄, pouvant être identiques ou différents, sont choisis dans le groupe constitué par l'hydrogène, les métaux des groupes principaux 1, 2 et 3 de la Classification Périodique des Eléments tels que Na, K, l'ammonium substitué ou non substitué et les composés combinés à l'ammonium dérivés de l'éthylènediamine,
X est une liaison simple ou bien est choisi dans le groupe constitué par les groupes (CH₂)_{1 à 6}, (CH₂)_{1 à 5} et amidino,
R₁ est choisi dans le groupe constitué par H, OH, NH₂, -CH₃, et
R₂ est choisi dans le groupe constitué par :

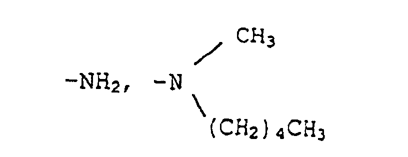

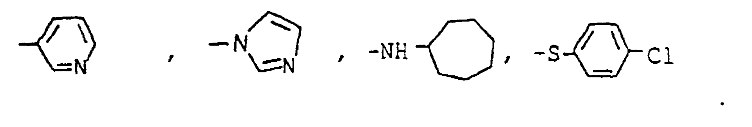

3. Utilisation selon la revendication 2,
**caractérisée en ce que**
les bisphosphonates sont choisis dans le groupe constitué par l'acide aminohydroxyméthylidènebiphosphonique,
l'acide 2-amino-1-hydroxyéthylidène-1,1-biphosphonique,
l'acide 3-amino-1-hydroxypropylidène-1,1-biphosphonique,
l'acide 4-amino-1-hydroxybutylidène-1,1-biphosphonique,
l'acide 6-amino-1-hydroxyhexylidène-1,1-biphosphonique,
l'acide aminométhylènebiphosphonique,
l'acide 3-méthylpentylamino-1-hydroxypropylidène-1,1-biphosphonique,
l'acide 2-(3-pyridinyl)-1-hydroxyéthylidène-1,1-biphosphonique,
l'acide 1-hydroxy-2-(imidazol-1-yl)-éthylidène-1,1-biphosphonique,
l'acide cycloheptylaminométhylènediphosphonique,
l'acide 4-chlorophényl-thiométhylène-1,1-biphoshphonique ainsi que leurs dérivés.

4. Utilisation selon l'une quelconque des revendications précédentes pour la thérapie et la prophylaxie de l'acné vulgaire, de la tuberculose chez l'Homme et l'animal, de la lèpre et d'autres mycobactéries chez l'Homme et l'animal, de la paratuberculose des animaux, d'entérites à Campylobacter chez l'Homme et l'animal, de Helicobacter Pylori et des *Chlamydia* en vue de la prophylaxie ou dans le cadre d'une thérapie de maladies cardio-vasculaires, en particulier de la maladie coronarienne.

5. Utilisation selon l'une quelconque des revendications 1 à 3 pour la thérapie par éradication de bactéries et de virus.

6. Utilisation selon la revendication 5 pour l'éradication de Helicobacter Pylori et des Chlamydia.

7. Utilisation selon la revendication 5 pour l'éradication de papillomavirus en vue de la prophylaxie de tumeurs, en particuliers de tumeurs des organes sexuels causées par des papillomavirus chez les Hommes, l'éradication des herpèsvirus, l'éradication des herpèsvirus 8 humains en vue du traitement du sarcome de Kaposi, l'éradication de cytomégalovirus préalablement à des transplantations, l'éradication de virus Epstein-Barr préalablement à une transplantation et en vue de la prophylaxie de tumeurs associées aux virus Epstein-Barr, l'éradication des virus des hépatites en vue du traitement de maladies hépatiques chroniques et en vue de la prophylaxie de tumeurs hépatiques et de cirrhoses hépatiques, l'éradication des virus Coxsackie dans le cas de cardiomyophaties, l'éradication des virus Coxsackie chez les patients souffrants de diabète sucré, l'éradication de virus de l'immunodéficience chez l'Homme et l'animal, le traitement d'infections opportunistes chez les patients souffrant du SIDA, le traitement d'inflammations d'origine virale de l'appareil respiratoire (papillome du larynx, hyperplasie, rhinite, pharyngite, bronchite, pneumonies), du foie et du système biliaire (hépatite, cholangite, carcinome hépatocellulaire), du tissu lymphatique (mononucléose, lymphadénite), du système hématopoïétique, de la peau (verrues, dermatite, herpès labial, boutons de fièvre, herpès zoster, zona), des muqueuses (papillomes, papillome de la conjonctive, hyperplasies, dysplasies), du système cardio-vasculaire (artérite, myocardite, endocardite, péricardite), de l'appareil réno-urinaire, des organes sexuels (lésions anogénitales, verrues, verrues génitales, condylomes acuminés, dysplasies, papillomes, dysplasies cervicales, *Condylomata acuminaka, Epidermodysplasia verruciformis*), des organes du mouvement (myosite, myalgies) à l'exception du SIDA et des inflammations provoquées par le SIDA ainsi que de ses maladies opportunistes, à savoir la dégénérescence des tissus conjonctifs.

8. Utilisation selon la revendication 7 pour l'éradication du virus de l'hépatite C.

9. Utilisation selon l'une quelconque des revendications précédentes, **caractérisée en ce que** le médicament contient en outre un véhicule acceptable sur le plan pharmaceutique.

10. Utilisation selon l'une quelconque des revendications précédentes en tant qu'additif pour des vaccins.
